## Europäisches Patentamt

## European Patent Office

## Office européen des brevets

(11) Veröffentlichungsnummer: **0 003 318**
**B1**

(12) # EUROPÄISCHE PATENTSCHRIFT

(45) Veröffentlichungstag der Patentschrift:
28.10.81

(51) Int. Cl.³: **C 12 N 1/04, C 12 N 1/14,**
**C 12 N 1/20, A 23 C 19/032,**
**C 12 C 11/00**

(21) Anmeldenummer: 79100136.5

(22) Anmeldetag: 18.01.79

(54) Verfahren zum Aufbereiten von Lebensmitteln und Präparat zum Schützen von zur Lebensmittelaufbereitung dienenden Mikroorganismen.

(30) Priorität: 27.01.78 LU 78955
02.01.79 LU 80748

(73) Patentinhaber: CHIMICASA GMBH, Wiesentalstrasse 81,
CH-7000 Chur (CH)

(43) Veröffentlichungstag der Anmeldung:
08.08.79 Patentblatt 79/16

(72) Erfinder: Wolf, Erich, Dr., Florianstrasse 19,
D-5066 Overath-Maria-linden (DE)
Erfinder: Lembke, Andreas, Prof.Dr.med., Eutiner
Strasse 1, D-2420 Eutin-Sielbeck (DE)
Erfinder: Deininger, Rolf, Dr., Fürst-Pückler-Strasse 44,
D-5000 Köln 41 (DE)

(45) Bekanntmachung des Hinweises auf die Patenterteilung:
28.10.81 Patentblatt 81/43

(84) Benannte Vertragsstaaten:
BE CH DE FR GB IT NL SE

(74) Vertreter: Hach, Hans Karl, Dr., Tarunstrasse 23,
D-6950 Mosbach-Waldstadt (DE)

(56) Entgegenhaltungen:

GB-B-560 800
CHEMICAL ABSTRACTS, Vol. 76, Nr. 4,
24. Januar 1972, Zusammenfassung Nr. 177715d,
Columbus, Ohio, USA,
JAIN S.R. et al.:
«Antibacterial activity of some essential oils and their
combinations»,
Seite 223, linke Spalte
CHEMICAL ABSTRACTS, Vol. 55, Nr. 4,
20. Februar 1961, Zusammenfassung Nr. 3726e,
Columbus, Ohio, USA,
J.C. MARUZZELLA et al.:
«Antibacterial activity of essential oil vapors»,
Spalte 3726
CHEMICAL ABSTRACTS, Vol. 77, Nr. 1,
3. Juli 1972, Zusammenfassung Nr. 729j,
Columbus, Ohio, USA,
HARADA MASATOSHI et al:
«Pharmacological studies on Chinese Cinnamon. I. Central effects of cinnamaldehyde» ,
Seite 75, rechte Spalte
CHEMICAL ABSTRACTS, Vol. 75, Nr. 23
6. Dezember 1971, Zusammenfassung Nr. 139241w,
Columbus, Ohio, USA,

(56) Entgegenhaltungen:

E.M. BOIYD et al.:
»Autumn-enhanced mucotropic action of inhaled terpenes and related volatile agents»,
Seite 197, linke Spalte
CHEMICAL ABSTRACTS, Vol. 81, Nr. 13,
30. September 1974, Zusammenfassung Nr. 78099f,
Columbus, Ohio, USA,
D. GOECKERITZ et al.:
»Carvone-camphor-type terpenes and terpene derivatives. Their antimicrobial and verminoxious properties»,
Seite 478, linke Spalte
CHEMICAL ABSTRACTS, Vol. 74, Nr. 11,
15. März 1971, Zusammenfassung Nr. 51906k,
Columbus, Ohio, USA,
E.M. BOYD:
«Effect of inhalation of citral and geraniol on the output and composition of respiratory tract fluid»,
Seite 202, rechte Spalte
UNLISTED DRUGS, Vol. 20, Nr. 9, Seite 129,
rechte Spalte «313, preparation», September 1968,
& Wien Med.Wschr. 118:346, 13. April 1968
Heinz A. Hoppe, Drogenkunde, Bd 1
(Anjiospermen) 8. Auflage (1975)
S. 288–291, 452–453, 838

### Verfahren zum Aufbereiten von Lebensmitteln und
### Präparat zum Schützen von zur Lebensmittelaufbereitung dienenden Mikroorganismen.

Die Erfindung betrifft ein Verfahren zum Aufbereiten von Lebensmitteln unter Einwirkung von Mikroorganismen, bei dem eine lebens- und vermehrungsfähige Kultur der Mikroorganismen dem aufzubereitenden Lebensmittel angeimpft wird und für diese, für die Dauer der angestrebten Einwirkung durch Zugabe von Nährstoffen und/oder Einstellen von Temperatur, Feuchtigkeit und/oder pH-Wert der angestrebten Einwirkung entsprechende Lebensbedingungen geschaffen und aufrechterhalten werden.

Die industrielle Lebensmittelaufbereitung, wie zum Beispiel Vergärung, Verkäsung, Fermentierung, erfolgt unter Mitwirkung von Mikroorganismen, nämlich Bakterien, Hefen, Pilzen oder Algen. Diese Mikroorganismen unterliegen in den Kulturen, in denen sie bereitgehalten werden, und auch während der Einwirkung auf die Lebensmittel dem Befall von Phagen und Viren, wodurch die mit den Mikroorganismen angestrebten Umsetzungsprozesse gestört werden können.

Aufgabe der Erfindung ist es, diese Störungen zu unterbinden.

Die Erfindung ist dadurch gekennzeichnet, dass die Mikroorganismen gegen Befall von Phagen und Viren geschützt werden, indem eines oder mehrere der aus Gewürzpflanzen gewinnbaren ätherischen Öle Schwarzer Pfeffer-Öl, Zimtblüten-Öl, Cardamom-Öl, Linallylacetat, Zimtaldehyd, Safrol, Carvon und cis/trans Citral in einer Menge von 1 bis 1000 mg (Milligramm) ätherisches Öl auf 10 kg (Kilogramm) Lebensmittel zugesetzt, untermischt und zur Einwirkung gebracht wird.

Es ist bekannt, dass die nach der Erfindung einzusetzenden ätherischen Öle toxische Wirkung auf Mikroorganismen ausüben, wie dies beispielsweise in CHEMICAL ABSTRACTS, Vol. 76, Nr. 4, 24. Januar 1972, Zusammenfassung Nr. 17715d für Schwarzer Pfeffer-Öl vorveröffentlicht ist. Aus GB-PS 560 800 ist vorbekannt, dass bicyclische Terpene und deren Derivate, insbesondere Kampfer-Öl und Bornylacetat einer Kultur aus mehreren Hefemutanten zuzusetzen und einen Mutanten auszulesen, der dabei einen Wachstumsvorteil erfährt. Diese Kultur ist aber nicht kontaminiert, deshalb findet keine Vireninaktivierung. Die anschliessend ausgelesene, isolierte Kultur wird ohne Terpen eingesetzt, daher findet auch bei der isolierten Kultur keine Vireninaktivierung durch Terpen statt – sie ist nach dieser Vorveröffentlichung auch nicht beabsichtigt.

Die nach der Erfindung einzusetzenden ätherischen Öle zeigen eine viruzide Wirkung (das heisst eine virenschädigende Wirkung) in einer Konzentration, die eine oder mehrere Zehnerpotenz niedriger liegt, als diejenige Konzentration, bei der diese ätherischen Öle toxische Wirkungen auf Mikroorganismen und andere lebende Zellen zeigen. Diese breite Spanne bietet einen bei der Dosierung vorteilhaften Toleranzspielraum, innerhalb dessen die angestrebte viruzide Wirkung erzielbar ist, ohne dabei Gefahr zu laufen, dass gleichzeitig die Mikroorganismen geschädigt werden.

Da diese ätherischen Öle aus Gewürzpflanzen gewonnen werden können, die sich für die menschliche und tierische Ernährung seit vielen Jahren bewährt und in der in Frage kommenden Dosierung als unschädlich erwiesen haben, ist auch sichergestellt, dass die nach der Erfindung einzusetzenden Mengen der ätherischen Öle keine schädigende Wirkung beim Verzehr der so behandelten Lebensmittel verursachen können.

Da die angestrebte viruzide Wirkung bei verhältnismässig geringem Zusatz der ätherischen Öle erzielbar ist, ist auch eine geschmackliche Beeinträchtigung der Lebensmittel durch die ätherischen Öle nicht in Kauf zu nehmen.

Nach erfolgter Animpfung vermehren sich die Mikroorganismen und man kann das zum Schutz dieser Gesamtmenge an Mikroorganismen erforderliche ätherische Öl von vornherein dem Lebensmittel zusetzen, man kann aber auch das ätherische Öl in zeitlich aufeinanderfolgenden Portionen entsprechend der Zunahme der Mikroorganismenpopulation zusetzen.

Bei der Aufbereitung von Milchprodukten unter Verwendung von Milchsäurestreptokokken und bei der Vergärung von Bierwürze unter Verwendung einer von Saccaromyces cerevisiae abgeleiteten Hefe beispielsweise, können die eingesetzten Mikroorganismen durch eines oder mehrere der oben genannten ätherischen Öle vor Virenbefall geschützt werden.

Die Erfindung betrifft ferner ein Präparat zum Schützen von zur Lebensmittelaufbereitung dienenden Mikroorganismen gegen Befall von Phagen und Viren, gekennzeichnet durch eines oder mehrere der aus Gewürzpflanzen gewinnbaren ätherischen Öle Schwarzer Pfeffer-Öl, Zimtblüten-Öl, Cardamom-Öl, Linallylacetat, Zimtaldehyd, Safrol, Carvon und cis/trans Citral eingemischt in eine Trägersubstanz, insbesondere in 1,2 Propandiol im Mischungsverhältnis 1 : 10 bis 1 : 1000.

Die eingesetzten ätherischen Öle können aus Gewürzpflanzen durch Wasserdampfdestillation gewonnen werden wie folgt: Schwarzer Pfeffer-Öl aus den Fruchtkörnern des Piper nigrum; Zimtblüten-Öl aus den Blüten der Cinnamonum Cassia; Cardamom-Öl aus dem Samen der Elettaria Cardamomum; Linallylacetat aus den Blüten der Lavandula; Zimtaldehyd aus der Rinde des Cinnamonum ceylanicum; Safrrol aus der Wurzel des Sassafras; Carvon aus der Frucht des Carum carvi und cis/trans Citral aus den Blättern der Cymbopogon citratus.

Man kann statt dieser natürlichen ätherischen Öle auch mit diesen identische synthetische ätherische Öle einsetzen, sofern solche zur Verfügung stehen. Bevorzugt werden aber die aus den Gewürzpflanzen gewonnenen natürlich ätherischen Öle.

## Beispiel 1

Zur Herstellung von Joghurt werden 100 l (Liter) Kuhmilch sterilisiert und dann auf 40 °C (Celsius) eingestellt. Dann werden 500 mg Schwarzer Pfeffer-Öl der Milch zugesetzt, durch Rühren gut untermischt und dann die Milch mit einer Reinkultur der Milchsäurebakterien lactobacillus bulgaricus angeimpft. Der Ansatz bleibt dann bei 40 °C geschützt vor äusserer Einwirkung 12 Stunden stehen, bis sich Joghurt gebildet hat.

## Beispiel 2

100 l fertige, abgefilterte Bierwürze werden auf 10 °C eingestellt. In diese Bierwürze werden 500 mg Schwarzer Pfeffer-Öl eingemischt, dann wird diese Bierwürze mit einer reinen Hefekultur, abgeleitet von Saccaromyces cerevisiae, angeimpft und bleibt bei 10 °C, geschützt vor äusserer Einwirkung, 8 bis 10 Tage stehen. Dann wird das nun fertige Bier von der Hefe, die sich inzwischen abgesetzt hat, abgezogen und auf Fässer abgefüllt.

## Beispiel 3

100 l fertige, abgefilterte Bierwürze werden auf 10 °C eingestellt. In diese Bierwürze werden 80 mg Schwarzer Pfeffer-Öl eingemischt. In 100 g (Gramm) reine Hefekultur, die abgeleitet ist von Saccaromyces cerevisiae, wird 1 mg Schwarzer Pfeffer-Öl eingemischt und 15 Minuten stehengelassen. Dann werden mit dieser Hefeportion die 100 l Bierwürze angeimpft, die dann bei 10 °C, geschützt vor äusserer Einwirkung, 8 bis 10 Tage stehenbleiben. Währenddessen werden 5 Stunden, 10 Stunden, 12 Stunden und 14 Stunden nach der Animpfung je eine Portion umfassend 2480 mg Schwarzer Pfeffer-Öl, in die gärende Bierwürze eingemischt. Nachdem die letzte Portion eingemischt ist, lässt man das gärende Bier ruhig stehen, so dass es ausgärt, die Hefe sich absetzt und das fertige Bier dann von der Hefe abgezogen und auf Fässer gefüllt werden kann. Es sind insgesamt 10 000 mg ätherisches Öl (Schwarzer Pfeffer-Öl) zugesetzt worden.

Bei diesen Beispielen wird durch das zugesetzte ätherische Öl schädigender Einfluss von Viren beziehungsweise Phagen unterbunden, ohne dass aufgrund der geringen Menge des zugesetzten ätherischen Öls geschmackliche oder sonstige Beeinträchtigungen in Kauf genommen werden müssen.

Die angegebenen Beispiele sind abänderbar, indem man anstelle des ätherischen Öls Schwarzer Pfeffer-Öl die gleiche Menge eines anderen erfindungsgemässen ätherischen Öls oder eine Mischung aus mehreren dieser ätherischen Öle einsetzt. Man kann auch die eingesetzte Menge des ätherischen Öls abändern im Rahmen der in den Ansprüchen angegebenen Spannen. In allen diesen Fällen wird eine viruzide Wirkung erzielt, ohne dass eine geschmackliche oder sonstige Beeinträchtigung dafür in Kauf genommen werden muss.

## Beispiel 4

Durch Wasserdampfdestillation aus Schwarzem Pfeffer wird Schwarzer Pfeffer-Öl gewonnen. Das Schwarzer Pfeffer-Öl wird im Gewichtsverhältnis 1 : 50 in 1,2-Propandiol gelöst. Die Lösung wird bei 121 °C während 50 Minuten im Autoklaven sterilisiert. Das so gewonnene Präparat wird dem durch Mikroorganismen aufzubereitenden Lebensmittel zugesetzt im Gewichtsverhältnis 50 mg bis 5,0 g Präparat auf 10 kg Lebensmittel und das entspricht 1 bis 100 mg ätherisches Öl auf 10 kg Lebensmittel.

Das Beispiel 4 ist abänderbar, indem man anstelle Schwarzer Pfeffer-Öl die gleiche Menge eines anderen erfindungsgemässen ätherischen Öls oder eine Mischung mehrerer dieser ätherischen Öle einsetzt. Man kann das Beispiel 4 auch dahingehend abändern, dass man das Gewichtsverhältnis ätherisches Öl zu 1,2-Propandiol im Bereich zwischen 1 : 10 und 1 : 1000 abändert. Dann muss man aber bei der Zugabe des Präparates in das Lebensmittel eine andere Menge einsetzen, so dass wieder die angestrebte Konzentration an ätherischem Öl 1 bis 100 mg ätherisches Öl auf 10 kg Lebensmittel erzielt wird.

Das Präparat nach Beispiel 4 beziehungsweise nach den Abänderungen dient dazu, dass ätherische Öl den Lebensmitteln zuzusetzen, und kann auch in Verbindung mit den Beispielen 1, 2 und 3 eingesetzt werden.

## Patentansprüche

1. Verfahren zum Aufbereiten von Lebensmitteln unter Einwirkung von Mikroorganismen, bei dem eine lebens- und vermehrungsfähige Kultur der Mikroorganismen dem aufzubereitenden Lebensmittel angeimpft wird und für diese, für die Dauer der angestrebten Einwirkung durch Zugabe von Nährstoffen und/oder Einstellen von Temperatur, Feuchtigkeit und/oder pH-Wert der angestrebten Einwirkung entsprechende Lebensbedingungen geschaffen und aufrechterhalten werden, dadurch gekennzeichnet, dass die Mikroorganismen gegen Befall von Phagen und Viren geschützt werden, indem eines oder mehrere der aus Gewürzpflanzen gewinnbaren ätherischen Öle Schwarzer Pfeffer-Öl, Zimtblüten-Öl, Cardamom-Öl, Linallylacetat, Zimtaldehyd, Safrol, Carvon und cis/trans Citral in einer Menge von 1 bis 1000 mg (Milligramm) ätherisches Öl auf 10 kg (Kilogramm) Lebensmittel zugesetzt, untermischt und zur Einwirkung gebracht wird.

2. Verfahren nach Anspruch 1, dadurch gekennzeichnet, dass dem aufzubereitenden Lebensmittel das ätherische Öl mit Beginn der Einwirkung der Mikroorganismen zugesetzt wird.

3. Verfahren nach Anspruch 1, dadurch gekennzeichnet, dass der Mikroorganismenkultur, bevor diese den Lebensmitteln angeimpft wird, ein Teil des ätherischen Öls zugesetzt wird und der Rest des ätherischen Öls den mit den Mikroorganismen angeimpften Lebensmitteln, und zwar in zeitlich aufeinanderfolgenden Portionen, verteilt über die Dauer der Einwirkung der Mikroorganismen, zugesetzt wird.

4. Verfahren nach Anspruch 1, 2 oder 3, gekennzeichnet durch die Anwendung bei der Aufbereitung von Milchprodukten unter Verwendung von Milchsäurestreptokokken.

5. Verfahren nach Anspruch 1, 2 oder 3, gekennzeichnet durch die Anwendung bei der Vergärung von Bierwürze unter Verwendung einer von Saccaromyces cerevisiae abgeleiteten Hefe.

6. Präparat zum Schützen von zur Lebensmittelaufbereitung dienender Mikroorganismen gegen Befall von Phagen und Viren, nach Anspruch 1, gekennzeichnet durch eines oder mehrerer der aus Gewürzpflanzen gewinnbaren ätherischen Öle Schwarzer Pfeffer-Öl, Zimtblüten-Öl, Cardamom-Öl, Linallylacetat, Zimtaldehyd, Safrol, Carvon und cis/trans Citral eingemischt in eine Trägersubstanz im Mischungsverhältnis 1 : 10 bis 1 : 1000, wobei die Trägersubstanz in das Lebensmittel gut einmischbar und als unschädlicher Lebensmittelzusatz geeignet ist.

7. Präparat nach Anspruch 6, gekennzeichnet durch 1,2-Propandiol als Trägersubstanz.

**Revendications**

1. Procédé de préparation d'aliments par l'action de micro-organismes, à savoir un procédé dans lequel une culture dans laquelle des micro-organismes peuvent vivre et se multiplier est introduite par inoculation dans les aliments à préparer et dans lequel il est créé et maintenu pour cette culture et pour la durée de l'action recherchée, des conditions de vie correspondant à une telle action, ce résultat s'obtenant par une addition de substances nutritives et/ou par le réglage de la température, de l'humidité et/ou par la valeur du pH, caractérisé par le fait que les micro-organismes sont protégés contre l'attaque des phages et des virus tandis qu'une ou plusieurs huiles essentielles, extrayables de plantes aromatiques, à savoir les suivantes: huile de Piper nigrum (poivre noir), essence de fleurs de cassie, essence de cardamone, acétate de linalyle, aldéhyde cinnamique, Safrol, carvone, citral à isomérie cis/trans, en quantité représentant 1 à 1000 mg (milligramme) d'huile essentielle par 10 kg (kilogramme) d'aliment, sont ajoutées et mélangées et entrent en action.

2. Procédé conforme à la revendication 1, caractérisé par le fait que c'est au début de l'action des micro-organismes que l'huile essentielle est ajoutée à l'aliment à préparer.

3. Procédé, conforme à la revendication 1, caractérisé par le fait qu'une partie de l'huile essentielle est ajoutée à la culture de micro-organismes avant que cette culture soit introduite dans les aliments par inoculation, tandis que l'huile essentielle restante est ajoutée aux aliments ensemencés de micor-organismes par inoculation, à savoir en quantités successives, réparties sur toute la durée de l'action desdits micro-organismes.

4. Procédé conforme à la revendication 1, 2 ou 3, caractérisé par le fait que dans la préparation des aliments susdite, on utilise des produits du lait ainsi que des streptocoques de l'acide lactique.

5. Procédé, conforme aux revendications 1, 2 ou 3, caractérisé par le fait que dans la fermentation, on utilise du moût de brasserie et une levure dérivée du «saccharomyces cerevisiae».

6. Préparation, permettant de protéger contre l'attaque de phages et de virus, les micro-organismes utilisés dans la préparation des aliments, à savoir une préparation conforme à la revendication 1, caractérisée par le fait que pour la faire, on utilise une ou plusieurs des huiles essentielles suivantes, extrayables de plantes aromatiques, à savoir les suivantes: huile de Piper nigrum (poivre noir), essence de fleurs de cassie, huile de cardamone, acétate de linalyle, aldéhyde cinnamique, Safrol, carvone, citral à isomérie cis/trans, mélangées à une substance porteuse, dans un rapport de mélange, compris entre 1 : 10 et 1 : 1000, la substance porteuse étant bien miscible dans l'aliment susdit à l'égard duquel elle se révèle un additif inoffensif convenant parfaitment bien pour un tel usage.

7. Préparation conforme à la revendication 6, caractérisée par le fait que la substance porteuse utilisée à la fin susdite est le 1,2-propanediol.

**Claims**

1. Process for the preparation of foods under the action of microorganisms in which a viable, propagatable culture of microorganisms is inoculated into the food to be prepared and by adding nutrients and/or adjusting the temperature, humidity and/or pH value the living conditions corresponding to the sought action are created and maintained for the same throughout the action period, characterized in that the microorganisms are protected against attacks by phages and viruses in that one or more of the ethereal oils obtainable from spice plants, black pepper oil, cassia oil, cardamom oil, linalyl acetate, cinnamaldehyde, safrole, carvone and cis/trans citral are added in a quantity of 1 to 1000 mg of ethereal oil per 10 kg of food, mixed and reacted.

2. Process according to claim 1, characterized in that the ethereal oil is added to the food to be prepared when the microorganisms start to act.

3. Process according to claim 1, characterized in that part of the ethereal oils is added to the microorganism culture before it is inoculated into the foods and the remainder of the ethereal oil is added to the foods inoculated with the microorganisms in portionswise manner spaced over the duration of the action of the microorganisms.

4. Process according to claims 1, 2 or 3, characterized by use in the preparation of milk products using lactic acid streptococci.

5. Process according to claims 1, 2 or 3, characterized by use in the attenuation of wort using yeast derived from Saccaromyces cerevisiae.

6. Product for protecting microorganisms used in the preparation of foods against the attack of phages and viruses according to claim 1, characterized by one or more of the ethereal oils obtainable from spice plants black pepper oil, cassia oil, cardamom oil, linalyl acetate, cinnamaldehyde, safrole, carvone and cis/trans citral mixed into a carrier substance in a mixing ration 1 : 10 to 1 : 1000, the carrier substance being readily mixable with the food and suitable as a harmless food additive.

7. Product according to claim 6, characterized by propylene glycol as the carrier substance.